# NEUE EUROPÄISCHE PATENTSCHRIFT

(11) **EP 0 891 781 B2**
(45) Veröffentlichungstag und Bekanntmachung des Hinweises auf die Entscheidung über den Einspruch: **20.02.2013**
(45) Hinweis auf die Patenterteilung: 24.09.2003
(21) Anmeldenummer: 98113385.3
(22) Anmeldetag: 17.07.1998
(51) Int. Cl.: A61L 2/06, A61L 2/07, A23L 3/04

(54) **Verfahren zum Sterilisieren von in flexiblen Folienverpackungen befindlichem Sterilisiergut und Vorrichtung zur Durchführung des Verfahrens**
Process for the sterilisation of acticles contained in flexible foil packages and device for carrying out such process
Procédé de stérilisation d'articles contenu dans des emballages en feuilles flexibles et dispositif pour mettre en oeuvre ce procédé

(30) Priorität: 18.07.1997 DE 19730901
(43) Veröffentlichungstag der Anmeldung: 20.01.1999
(73) Patentinhaber: Fresenius AG, 61352 Bad Homburg v.d.H. (DE)
(72) Erfinder: Knierbein, Bernd, Dr., 61267 Neu-Anspach (DE); Kessler, Barbara, Dr., 61476 Kronberg (DE)
(74) Vertreter: Mai, Oppermann & Partner i.L.

(56) Entgegenhaltungen:
- EP-A- 0 691 272
- WO-A1-88/04143
- CH-A- 348 786
- DE-A- 2 339 226
- NL-C- 91 848
- US-A- 3 634 099
- US-A- 5 301 603

## Beschreibung

Die Erfindung betrifft ein Verfahren zum Sterilisieren von in flexiblen Standbodenbeuteln befindlichem Sterilisiergut, insbesondere mit einer Lösung zur enteralen Ernährung befüllte Standbodenbeutel.

Zur Sterilisation finden Autoklaven Verwerdung in denen der Sterilisiervorgang in drei Phasen abläuft, nämlich dem Steigen, Halten und Fallen. Bekannt sind periodisch und kontinuierlich arbeitende Autoklaven, die sich in der Arbeitsweise und Bauart unterscheiden. Periodisch arbeitende Autoklaven werden mit einer Charge beschickt und verschlossen, worauf die drei Phasen des SterilisationsProzesses nacheinander durchlaufen werden. Mit einer neuen Charge kann erst nach dem Entleeren des Autoklaven begonnen werden. Der zusätzliche Zeitaufwand durch die Totzeiten während des Entleerens und Beschickens erweisen sich bei dem periodisch arbeitenden Autoklaven als nachteilig. Ein Vorteil liegt jedoch in den Variationsmöglichkeiten der Sterilisierbedingungen von Charge zu Charge. Die kontinuierlich arbeitenden Autoklaven sind hingegen bezüglich ihres automatischen Prozeßablaufes, der genauen Einhaltung der Sterilisationsbedingungen sowie ihrer Wirtschaftlichkeit den periodisch arbeitenden Autoklaven überlegen. So ist der Energiebedarf und der Wasserverbrauch deutlich niedriger.

Als kontinuierlich arbeitende Autoklaven sind die hydrostatischen Autoklaven bekannt, bei denen der Druck im Autoklavenraum durch eine entsprechend hohe Wassersäule kompensiert wird. Das Sterilisieret durchläuft in dem hydrostatischen Autoklaven eine erste Wassersäule zum Anheizen, eine Dampfphase mit konstanter Temperatur zum Sterilisieren und eine zweite Wassersäule zum Abkühlen. Da die hydrostatischen Autoklaven zur Erreichung von Sterilisationstemperaturen von über 100°C systembedingt eine sehr große Bauhöhe haben müssen, sind diese auch als Autoklaventürme bekannt. Es können auch mehrere Wassersäulen hintereinander geschaltet werden. (Lueger Bd. 16, Seiten 52, 53). Aus der US 3,634,099 sind solche Autoklaven bekannt, die zur Sterilisierung vom gefüllten Beuteln aus einem Aluminium- Kunststoff- Laminat eingesetzt werden.

Obwohl die hydrostatischen Autoklaven hinsichtlich des automatischen Prozeßablaufes und der Wirtschaftlichkeit große Vorteile bieten, werden sie teilweise für die Sterilisation von in flexiblen Folienverpackungen befindlichem Sterilisiergut, insbesondere zum Sterilisieren von in Folienbeuteln befindlichen Lösungen zur enteralen Ernährung, nicht eingesetzt. Dies liegt darin begründet, daß aufgrund des relativ geringen Stützdruckes, der im hydrostatischen Autoklaven erzeugt werden kann, die flexible Verpackung einer verhältnismäßig großen mechanischen Beanspruchung ausgesetzt ist. Während der Sterilisationsphase liegt in der Folienverpackung nämlich ein höherer Druck vor, als in der Dampfzone. Diese Druckdifferenz kann zu einer Dehnung der Verpackungsfolie führen. Zur Sterilisation von in flexiblen Folienverpackungen befindlichem Sterilisiergut werden daher teilweise die periodisch arbeitenden Autoklaven verwendet, in deren Druckbehälter ein entsprechender Stützdruck erzeugt werden kann.

Aus der WO 88/04143 ist ein Verfahren zum Sterilisieren von flüssigkeitsgefüllten Kunststoffbehältern bekannt, bei dem das Sterilisiergut in Aufnahmeinrichtungen durch den Autoklaven gefördert wird. Das Sterilisiergut liegt auf einem dünnen Flüssigkeitsfilm auf, der sich auf der Bodenplatte der Aufnahmeeinrichtungen ausbildet. Die Aufnahmeeinrichtungen weisen zwar seitliche Begrenzungen auf, sie sind jedoch nicht dazu bestimmt, die während der Sterilisation auf das Sterilisiergut wirkende Kräfte aufzunehmen. Für den Fall, dass flexible Folienverpackungen nach dem bekannten Verfahren sterilisiert werden sollen, sind Verformungen derselben daher nicht auszuschließen.

Der Erfindung liegt die Aufgabe zugrunde, ein Verfahren anzugeben, das eine kontinuierliche Sterilisation von in empfindlichen flexiblen Standbodenbeuteln befindlichem Sterilisiergut, insbesondere in Standbodenbeuteln befindlichen Lösungen zur enteralen Ernährung, anzugeben, das eine kontinuierliche Arbeitsweise erlaubt, wodurch die Wirtschaftlichkeit verbessert wird. Die Lösung dieser Aufgabe erfolgt erfindungsgemäß mit den Merkmalen des Patentanspruchs 1.

Die das Sterilisiergut enthaltenden Standbodenbeutel werden bei dem erfindungsgemäßen Verfahren in Stützeinrichtungen eingelegt und in den Stützeinrichtungen durch den hydrostatischen Autoklaven gefördert. Während der Sterilisation nehmen die Stützeinrichtungen die auf die Innenseite der Verpackung wirkenden Kräfte auf, so daß die Verpackungsfolie keinen größeren mechanischen Beanspruchungen ausgesetzt ist. Die Verpackungen können sich während der Sterilisation gegen die Stützeinrichtungen anlegen und daher nur geringfügig verformen. Im allgemeinen ist es ausreichend, wenn die Verpackungen von den Stützeinrichtungen nur teilweise umschlossen werden. Bei temperatur- und festigkeitsbeständigen Folienmaterialien, wie biaxial verstrecktem PET, treten selbst im Falle einer teilweisen Abstützung nur geringfügige Verstreckungen auf Bei anderen Materialien kommt es zu einer eingrenzbaren Verstreckung in den freien Bereichen. Um eine optimale Abstützung zu erhalten, sollten die Stützeinrichtungen an die Form der Standbodenbeutel angepaßt sein. Sie können als geschlossene Behälter oder auch Körbe ausgebildet sein.

Die Verpackungen werden zwischen einer Bodenplatte und einer Deckplatte der Stützeinrichtungen leicht klemmend fixiert. Die Boden- und Deckplatte können mit muldenförmigen Vertiefungen versehen sein, die der Außenkontur der Verpackungen entsprechen. Zur Verbesserung des Wärmeübergangs sind die Boden- und Deckplatte der Stützeinrichtung gelocht.

Obwohl in dem kontinuierlich arbeitenden Autoklaven nur ein geringer Stützdruck erzeugt werden kann, ist eine Sterilisation auch von in flexiblen Folienverpackungen befindlichem Sterilisiergut möglich. Mit dem erfindungsgemäßen Verfahren können daher Standbodenbeutel sterilisiert werden, die eine Lösung zur enteralen Ernährung enthalten. Der kontinuierliche Sterilisationsprozeß im hydrostatischen Autoklaven hat im Vergleich zum periodisch arbeitenden Autoklaven einen geringeren Energiebedarf und Wasserverbrauch. Auch ist der Platzbedarf bei hohen Sterilisationsleistungen geringer und die Überwachung des kontinuierlichen Sterilisationsprozesses ist einfacher. Der Sterilisationsprozeß kann direkt dem Abfüllprozeß nachgeschaltet werden. Lebensmittelprodukte, die UHT erhitzt abgefüllt werden, können unmittelbar nach dem Abfüllen noch warm dem Autoklaven zugeführt werden, so daß das Produkt geschont und ein Teil der Erwärmungsenergie eingespart wird.

Während die das Sterilisiergut enthaltenden Verpackungen durch den hydrostatischen Autoklaven gefördert werden, sind Kipp- und Drehbewegungen der Stützeinrichtungen von Vorteil. Dadurch wird eine verbesserte Durchmischung des Sterilisiergutes erreicht und ein konvektiver Temperaturausgleich im Produkt kann stattfinden, was bei schlecht wärmeleitenden Produkten vorteilhaft ist. Darüber hinaus werden durch die Kipp- oder Drehbewegungen der Stützeinrichtungen bei feststoffhaltigen Produkten Anbackungen vermindert oder vermieden. Bei kritischen Produkten ist auch eine kontinuierliche Drehung der Stützeinrichtungen während der gesamten Sterilisation von Vorteil. Hierbei müssen die Stützeinrichtungen jedoch so beschaffen sein, dass die Verpackungen ausreichend fixiert sind.

Im folgenden wird unter Bezugnahme auf die Zeichnungen ein Ausführungsbeispiel einer Vorrichtung zur Durchführung des Verfahrens näher erläutert.

Es zeigen:
Figur 1 den schematischen Aufbau des hydrostatischen Autoklaven,
Figur 2 eine Stützeinrichtung des hydrostatischen Autoklaven zur Aufnahme von das Sterilisiergut enthaltenden Standbodenbeuteln in schematischer Darstellung,
Figur 3 einen Schnitt durch die Stützeinrichtung von Figur 2 und
Figuren 4a - 4c verschiedene Ausführungsformen von in die Stützeinrichtung von Figur 2 passenden Folienbeuteln.

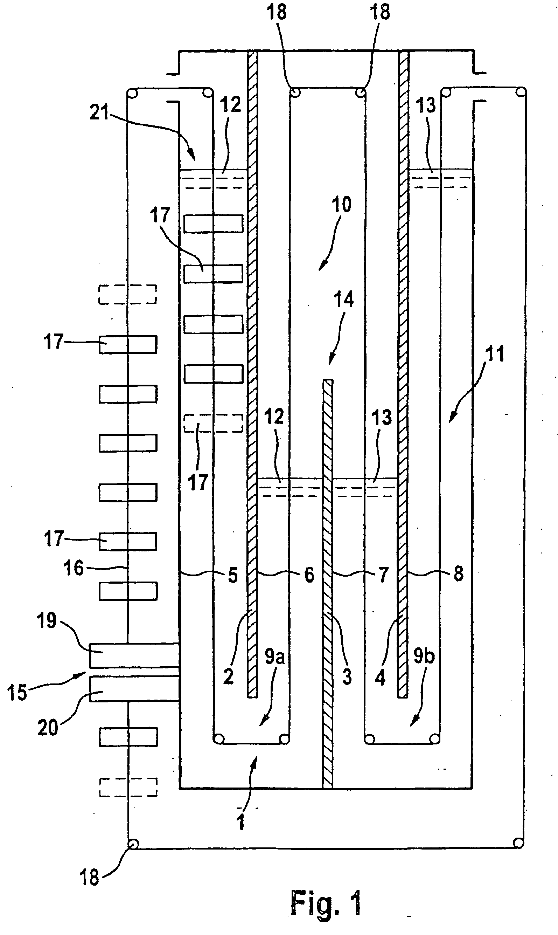

Figur 1 zeigt den schematischen Aufbau eines hydrostatischen Autoklaven. Der hydrostatische Autoklav weist einen Autoklavenraum 1 auf, der durch drei Trennwände 2, 3, 4 in vier Zonen 5 bis 8 unterteilt ist, die von den Folienbeuteln nacheinander durchlaufen werden. Die erste und zweite Zone 5, 6 bzw. die dritte und vierte Zone 7, 8 stehen über einen Durchlaß 9a, 9b am Boden des Autoklavenraums 1 in Verbindung, während die zweite und dritte Zone 6, 7 über einen Durchlaß 10 in der oberen Hälfte des Autoklavenraums in Verbindung stehen. Der Autoklavenraum 1 ist teilweise mit Wasser 11 gefüllt, so daß sich in der ersten und zweiten Zone 5, 6 eine erste Wassersäule 12 zum Anheizen und in der dritten und vierten Zone 7, 8 eine zweite Wassersäule 13 zum Abkühlen ausbildet. Oberhalb der Wassersäulen 12, 13 befindet sich in der zweiten und dritten Zone 6, 7 des Autoklavenraums die Dampfphase 14.

Zum Fördern des Sterilisiergutes durch die Wassersäulen 12, 13 und die Dampfphase 14 ist eine Fördereinrichtung 15 vorgesehen, die an Ketten 16 geförderte Stützeirichtungen 17 zur Aufnahme der Standbodenbeutel aufweist. Die Ketten 16 laufen auf Zahnrädern 18 entlang einer geschlossenen Förderstrecke. Die Stützeinrichtungen 17 sind in gleichbleibenden Abständen entlang der geschlossenen Förderstrecke angeordnet. Figur 1 zeigt nur einen Teil der Stützeinrichtungen.

An den Seiten des Autoklavenraums ist eine Beschickungseinrichtung 19 und eine Entnahmeeinrichtung 20 zum Abtransport der Folienbeutel vorgesehen. Die Folienbeutel werden zum Sterilisieren mit der Beschickungseinrichtung 19 in die Stützeinrichtungen 17 der Fördereinrichtung 15 eingelegt. Die Folienbeutel treten zum Anheizen in die erste Wassersäule 12 ein, wobei Temperatur der Folienbeutel an der Eintrittsstelle 21 am niedrigsten ist und in Förderrichtung kontinuierlich ansteigt. In der zweiten Zone 6 treten die Folienbeutel aus der ersten Wassersäule 12 aus und durchlaufen die Dampfphase 14, um dann in der dritten Zone 7 in die zweite Wassersäule 13 zum Abkühlen einzutreten wobei die Temperatur der Folienbeutel in Förderrichtung kontinuierlich abnimmt. In der vierten Zone 8 treten die Folienbeutel aus der zweiten Wassersäule 13 aus und werden zu dem unterhalb der Beschickungseinrichtung 19 angeordneten Entnahmeeinrichtung 20 gefördert, wo diese den Stützeinrichtungen 17 entnommen und abtransportiert werden.

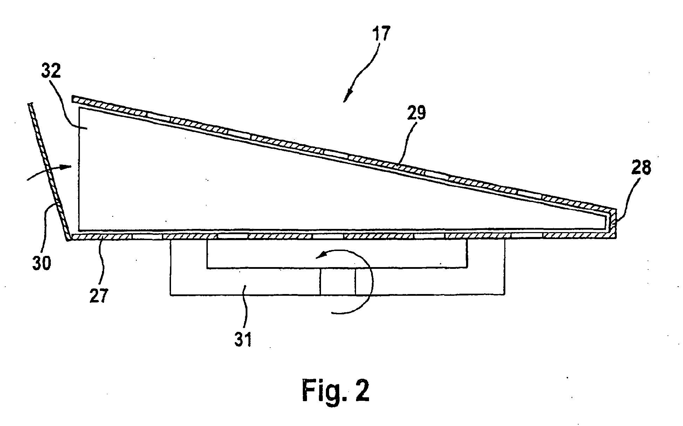

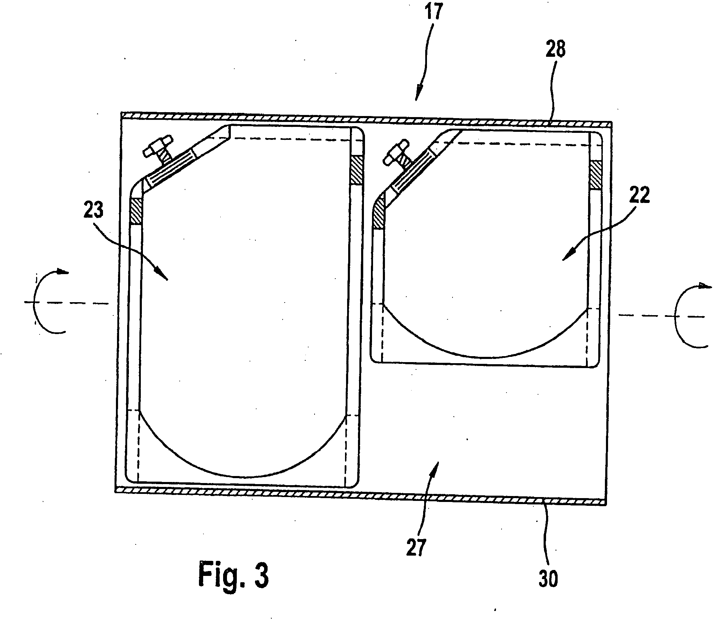

Die an den Ketten der Fördereinrichtung durch den Autoklavenraum geförderten Stützeinrichtungen zur Aufnahme der Folienbeutel werden nachfolgend anhand der Figuren 2 und 3 erläutert.

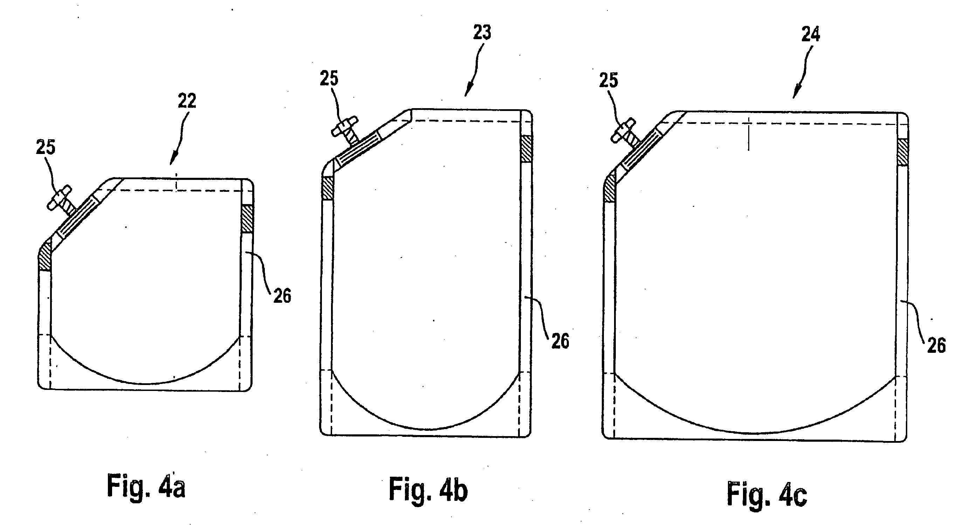

Die Stützeinrichtungen dienen zur Aufnahme der in den Figuren 4a bis 4c gezeigten Standbodenbeutel, die aufgrund ihrer Geometrie und der genügenden Foliensteifigkeit eine definierte Außenkontur besitzen. Die eine Lösung zur enteralen Ernährung enthaltenden Standbodenbeutel 22, 23, 24 mit einem Beutelvolumen von 500 ml, 1000 ml bzw. 1500 ml haben jeweils einen Port 25 zum Anschluß eines nicht dargestellten Überleitgerätes, der in die Beutelnaht 26 eingeschweißt ist

Jede Stützeinrichtung 17 weist eine rechteckförmige gelochte Bodenplatte 27 aus Metall auf, deren vorderer Rand 28 umgebogen ist. An dem umgebogenen Rand 28 der Bodenplatte 27 ist eine rechteckförmige gelochte Deckplatte 29 aus Metall unter Bildung eines Faches mit einem keilförmigen Querschnitt befestigt, wobei die Bodenplatte und die Deckplatte einen Winkel einschließen, der zwischen 5 und 15° liegt. Die Stützeinrichtung kann mit einer Klappe 30 verschlossen werden, die an der Bodenplatte 27 angelenkt ist und über einen nicht dargestellten Verriegelungsmechanismus verfügt. An den Seiten kann die Stützeinrichtung 17 offen sein. Es können aber auch Seitenplatten vorgesehen sein.

Die Bodenplatte 27 der Stützeinruchtung 17 ist an einem Träger 31 befestigt, der drehbar an den Ketten der Fördereinrichtung aufgehängt ist. Die Bewegung des Trägers 31 erfolgt mit einem nicht dargestellten Drehantrieb.

Der Standbodenbeutel 32 wird seitlich in die Stützeinrichtung 17 eingeschoben und die Klappe 30 wird geschlossen. Die Stützeinrichtung ist derart ausgebildet, daß der Folienbeutel mit seiner Unterseite flach auf der Bodenplatte 27 aufliegt und die Oberseite an der Deckplatte 29 anliegt. Da die Seitenteile des Folienbeutels während des Sterilisationsvorganges nur relativ gering belastet werden, ist hier eine Abstützung nicht erforderlich.

In die Stützeinrichtung können Folienbeutel unterschiedlicher Größe (Figuren 4a bis 4c) nebeneinanderliegend eingeschoben werden (Figur 3), da alle Beutelgrößen einschließlich Port einen ähnlichen Querschnitt im oberen Beutelbereich haben und auf ein den Beutel seitlich begrenzendes Gefache verzichtet werden kann. Insofern können ohne Umrüstarbeiten verschiedene Folienbeutel sterilisiert werden.

## Patentansprüche

1. Verfahren zum Sterilisieren von in flexiblen Standbodenbeuteln befindlichem Sterilisiergut, insbesondere mit einer Lösung zur enteralen Ernährung befüllte Standbodenbeutel, bei dem die das Sterilisiergut enthaltenden Standbodenbeutel (22, 23, 24) in einem hydrostatischen Autoklaven einer ersten Wassersäule (12) zum Anheizen zugeführt werden, durch eine Dampfphase (14) gefördert werden und einer zweiten Wassersäule (13) zum Abkühlen zugeführt werden, wobei die Standbodenbeutel in diese zumindest teilweise umschließende Stützeinrichtungen (17) einer Fördereinrichtung (15) eingelegt werden und in den Stützeinrichtungen mit der Fördereinrichtung durch den Autoklaven gefördert werden, **dadurch gekennzeichnet, dass** die das Sterilisiergut enthaltenden Standbodenbeutel in die Stützeinrichtungen (17) zwischen eine rechteckförmige, gelochte Bodenplatte (27) aus Metall, deren vorderer Rand (28) umgebogen ist, und eine rechteckförmige, gelochte Deckplatte (29) aus Metall seitlich eingeschoben werden, wobei die Bodenplatte und Deckplatte ein spitz zulaufendes Fach bilden und einen Winkel einschließen, der zwischen 5 und 15° liegt, so dass die Standbodenbeutel klemmend fixiert werden und während der Sterilisation die auf die Verpackungsfolie wirkenden Kräfte von den Stützeinrichtungen aufgenommen werden und wobei die Standbodenbeutel mit deren Unterseite flach auf der Bodenplatte (27) aufliegen und deren Oberseite an der . Deckplatte (29) anliegen.

2. Verfahren nach Anspruch 1, **dadurch gekennzeichnet, dass** die Stützeinrichtungen (17) gekippt und/oder gedreht werden, während die das Sterilisiergut enthaltenden Standbodenbeutel (22, 23, 24) durch den Autoklaven gefördert werden.

## Claims

1. Method for the sterilisation of sterilisable product in flexible stand-up bags, in particular stand-up bags filled with solutions for enteral nutrition, wherein the stand-up bags (22, 23, 24) containing the sterilisable product are fed in a hydrostatic autoclave to a first water column (12) for heating, conveyed through a steam phase (14) and fed to a second water column (13) for cooling, wherein the stand-up bags are placed into support devices (17) of a conveying device (15), said support devices at least partially surrounding said stand-up bags, and are conveyed in the support devices by the conveying device through the autoclave, **characterised in that** the stand-up bags containing the sterilisable product are pushed laterally into the support devices (17) between a rectangular, perforated base plate (27) made of metal, the front edge (28) whereof is bent over, and a rectangular, perforated cover plate (29) made of metal, wherein the base plate and the cover plate form an acutely tapered compartment and enclose an angle which lies between 5 and 15°, so that the stand-up bags are fixed in a clamping manner and, during the sterilisation, the forces acting on the packaging film are taken up by the support devices and wherein the stand-up bags lie with their underside flat on the base plate (27) and with their upper side lying adjacent to the cover plate (29)

2. The method according to claim 1, **characterised in that** the support devices (17) are tilted and/or rotated while the stand-up bags (22, 23, 24) containing the sterilisable product are being conveyed through the autoclave.

## Revendications

1. Procédé de stérilisation de produits à stériliser se trouvant dans des poches à fond plat flexibles, en particulier des poches à fond plat remplies d'une solution pour la nutrition entérale, dans le cadre duquel les poches à fond plat (22, 23, 24) contenant le produit à stériliser sont amenées dans un autoclave hydrostatique vers une première colonne d'eau (12) pour y être chauffées, sont transportées à travers une phase gazeuse (14) et sont amenées vers une deuxième colonne d'eau (13) pour y être refroidies, sachant que les poches à fond plat sont introduites dans des dispositifs de support (17) d'un dispositif de transport (15) entourant au moins en partie lesdites poches à fond plat et sont transportées dans les dispositifs de transport à l'aide du dispositif de transport à travers l'autoclave, **caractérisé en ce que** la poche à fond plat contenant le produit à stériliser est insérée latéralement dans les dispositifs de support (17) entre un plateau de fond (27) rectangulaire perforé en métal, dont le bord avant (28) est rabattu, et un plateau de couverture (29) rectangulaire perforé en métal, sachant que le plateau de fond et le plateau de couverture forment un compartiment convergeant en pointe et forment un angle compris entre 5 et 15° de sorte que les poches à fond plat sont fixées par serrage et que les forces exercées sur le film d'emballage sont absorbées au cours de la stérilisation par les dispositifs de support, sachant que les poches à fond plat reposent par leur côté inférieur à plat sur le plateau de fond (27) et par leur côté supérieur sur le plateau de couverture (29).

2. Procédé selon la revendication 1, **caractérisé en ce que** les dispositifs de support (17) sont basculés et/ou pivotés, tandis que les poches à fond plat (22, 23, 24) contenant le produit à stériliser sont transportées à travers l'autoclave.
